# EUROPEAN PATENT SPECIFICATION

(11) **EP 0 961 548 B1**
(45) Date of publication and mention of the grant of the patent: **19.09.2001**
(21) Application number: 97900682.2
(22) Date of filing: 20.01.1997
(51) Int. Cl.: A01N 63/04, C12N 1/16

(54) **METHOD AND COMPOSITION FOR FRUIT DISEASE CONTROL**
VERFAHREN UND ZUSAMMENSETZUNG ZUR BEKÄMPFUNG VON FRUCHTKRANKHEITEN
PROCEDE ET COMPOSITION POUR LA LUTTE CONTRE LES MALADIES AFFECTANT LES FRUITS

(43) Date of publication of application: 08.12.1999
(73) Proprietor: Agricultural Research Council, Hatfield, Pretoria (ZA)
(72) Inventor: DE KOCK, Selma, Louisa, Stellenbosch (ZA)
(74) Representative: Brewster, Andrea Ruth
(86) International application number: GB9700154
(87) International publication number: WO9831229

(56) References cited:
- WO-A-96/25039
- US-A- 5 244 680
- DATABASE CABA Commonwealth Agricultural Bureau International, Farnham Royal , Slough, GB; Accession-no. 96:61426, 1996 T.CHAND-GOYAL & R.A. SPOTTS: "Control of postharvest pear diseases using natural saprophytic yeast colonists and their combination with a low dosage of thiabendazole" XP002041898 & POSTHARVEST BIOLOGY AND TECHNOLOGY , vol. 7, no. 1/2, 1996, pages 51-64,
- DATABASE BIOSIS BIOSCIENCES INFORMATION SERVICE, PHILADELPHIA, PA, US ELAD Y ET AL: "Control of infection and sporulation of Botrytis cinerea on bean and tomato by saprophytic yeasts." XP002041899 & PHYTOPATHOLOGY 84 (10). 1994. 1193-1200. ISSN: 0031-949X,

## Description

This invention relates to a method of controlling or preventing fruit diseases. In particular, the invention relates to a method of controlling or preventing infection of fruit by plant pathogens, and to a biocontrol composition.

US 5,244,680-A, WO 96/25039A and CABA abstract No. 96:61426 (1996) disclose that strains of several Crypotococcus species, including certain specified strains of C.albidus, are highly effective in the control of post-harvest diseases in fruit caused by pathogens, e.g. Botrytis cinerea and Penicillium expansum. Database BIOSIS abstract No. 95:26305 (1995) discloses that some isolates of Cryptococcus albidus control Botrytis cinerea on whole bean and tomato plants under growth-room conditions.

According to one aspect of the invention there is provided a method of controlling or preventing infection of fruit by plant pathogens, which method includes applying to fruit, or to at least a part of a plant bearing the fruit, an effective amount of a biocontrol yeast strain of Cryptococcus albidus identified by Centraalbureau Voor Schimmelcultures (CBS) accession No. 604.94 which is non-pathogenic to the fruit and plant.

The yeast strain may be applied in the form of an aqueous suspension having a concentration of 1 X 10⁴ - 2 X 10⁸ propagules/cm³, preferably 1 X 10⁴ - 1 X 10⁸ propagules/cm³, e.g. 1 X 10⁸ propagules/cm³.

Preferably the yeast strain is applied at least once to the plant and/or at least once to the fruit after harvesting thereof. For example, the yeast strain may initially be applied to the plant at a fruit-bearing stage, e.g. shortly before harvesting, whereafter, after harvesting of the fruit, a further application of the yeast strain may be applied to the fruit to inhibit decay thereof by the plant or fruit pathogen. When applied to the fruit-bearing plant the suspension should be applied to the fruit and optionally to other parts of the plant, eg the leaves and stems in proximity to the fruit. The method of application may be by any suitable conventional means such as spraying. The aqueous suspension may be applied at a rate of between 500 and 1000 ℓ/hectare, eg about 600 ℓ/hectare. When the yeast strain is applied to the fruit after harvesting of the fruit, this may be effected by drenching the fruit in an aqueous suspension of the yeast strain.

By shortly before harvesting is meant a period of 7 - 2 days before harvesting.

Preferably, the method is employed against fungal plant or fruit pathogens such as Botrytis cinerea and Penicillium expansum.

The plants may be deciduous fruit cultivars, such as pear and apple cultivars, so that the fruit can thus be pears or apples.

According to a further aspect of the invention, there is provided a biocontrol composition which comprises, as active biocontrol agent, an effective amount of a yeast strain of Cryptococcus albidus identified by CBS accession No. 604.94 in combination with a biologically suitable carrier material.

The carrier material may be any suitable carrier material such as sterile distilled water or a suitable buffer solution, eg. 0,9M sodium chloride.

According to another aspect of the invention there is provided a Cryptococcus albidus yeast isolate or strain identified by Centraalbureau Voor Schimmelcultures (CBS) accession No. 604.94.

It will be appreciated that the Cryptococcus albidus yeast isolate or strain of the invention has various unique features and characteristics, such as its ribosomal DNA fingerprint, which distinguishes it from other yeast isolates or strains of Cryptococcus albidus.

The invention is now described with reference to the following non-limiting Examples illustrating methods of controlling or preventing infection of fruit by plant pathogens in accordance with the invention.

### EXAMPLE 1 - Laboratory Trials

Cryptococcus albidus yeast cultures (CBS accession No. 604.94) were grown for about 16 hours at 24°C in yeast extract peptone-dextrose broth to a mid-log phase. Cells of the yeast isolate cultures were harvested by centrifuging suspensions of the cultures at 6000X g for 20 minutes. The supernate was suspended in sterile, distilled water and the centrifugation step repeated. The supernate was then re-suspended in sterile, distilled water, to provide a biocontrol composition according to the invention and containing 1 X 10⁸ propagules/cm³.

Wounded pear fruit of the cultivar Packham's Triumph were inoculated with this biocontrol composition by pipetting 15 *µ*l of the composition onto each wound. Three hours later, 15 *µ*l of an aqueous suspension of a Penicillium expansum fungal culture, at a concentration of 10 000 conidia/cm³ was also pipetted onto each of the wounds. Wounded control fruit were inoculated in a similar fashion with sterile, distilled water and three hours later 15 *µ*l of the Penicillium expansum culture (concentration 10 000 conidia/cm³) was pipetted onto each wound of the control fruit. Treatments, including controls, were replicated six times each with sixty fruit per replicate. The test fruit and the control fruit were stored at a temperature of -0.5°C for one month and then stored at 20°C for one week. The number of fruit with decayed inoculation sites were then recorded.

The trials described above were then repeated with a Botrvtis cinerea fungal culture replacing the Penicillium expansum as the pathogenic test organism.

In the trials described above, all the replicates of the inoculated control fruit developed decay but the replicates of the test fruit inoculated with the Cryptococcus albidus composition remained substantially free of decay.

### EXAMPLE 2 - Confidential Field Trials

Fruit on ten Packham's Triumph pear trees in an orchard were sprayed one week before harvest with an aqueous suspension of a Crvptococcus albidus culture (CBS accession No. 604.94) having a concentration of 1 X 10⁸ propagules/cm³, using a knap-sack sprayer and ensuring that the suspension was applied to the fruit as well as to leaves and stems in proximity to the fruit. Approximately one litre of the suspension was applied to each tree, and precautions were taken to prevent spray-drift. No wetting-agents were added to the yeast suspension. Unsprayed fruit-bearing trees in the same orchard served as controls. At harvest, 10 cartons (15 kg each) of the initially sprayed fruit were again sprayed with the yeast suspension having a concentration of 1 X 10⁸ propagules/cm³, using an atomizer. The treated fruit as well as 10 cartons of the control-fruit were cold-stored at -0.5°C for three months and then stored at 20°C for one week before incidence of decay on the fruit was recorded.

The treatment with the yeast Cryptococcus albidus before and after harvest reduced the incidence of total decay by approximately 50% in comparison with the control fruit.

### EXAMPLE 3 - Further Trials

A Cryptococcus albidus culture (CBS accession No. 604.94) was cultivated in bulk in 6 000 litres of a molasses medium, obtained from National Chemical Products in South Africa. The Cryptococcus albidus culture was separated from the growth medium by means of a commercial separator to provide a Cryptococcus albidus concentrate. 200 litres of the Cryptococcus albidus concentrate was stored at - 0.5°C until required.

Prior to use, the Cryptococcus albidus concentrate was diluted to provided an aqueous suspension of the Cryptococcus albidus culture as described hereunder.

### Trial A - Pears treated in Orchard prior to harvesting

Packham's pear trees growing on 0.5 hectare in an orchard were sprayed two days before harvest with the aqueous suspension of the Cryptococcus albidus culture using a commercial spray applicator (50 litres of Cryptococcus albidus culture concentrate per 1 000 litres of water). A wetting agent (Triton X-100 - trade mark) was added to the aqueous suspension prior to spraying and care was taken thoroughly to wet all pears on the trees. A total volume of approximately 1 000 litres of the aqueous suspension was applied to the pears and parts of the trees in close proximity to the pears, i.e. approximately equivalent to 2 000 litres/hectare. The concentration of Cryptococcus albidus cells deposited on the pears was determined shortly after application. Mean cell counts were 380 cells/cm² of fruit surface. As mentioned above, treated pears were harvested two days after treatment. Pears from untreated trees from an adjacent orchard served as control pears.

After harvest, the pears were divided into groups and were treated as follows:

### Group 1

No post-harvest treatment was applied.

### Group 2

Pears were drenched in the aqueous suspension in a commercial drench (50 litres of Cryptococcus albidus concentrate per 1 000 litres of water) for 2 minutes in a packhouse.

### Group 3

Pears were sprayed with the aqueous suspension (50 litres of Cryptococcus albidus concentrate per 1 000 litres of water) on a packing line by means of an atomizer.

### Trial B - Pears not treated prior to harvesting

Packham's pear trees growing on 0.5 hectare in an orchard were harvested. After harvest, the pears were divided into groups and were treated as follows:

### Group 4

No post-harvest treatment was applied (control pears).

### Group 5

Pears were drenched in the aqueous suspension in a commercial drench (50 litres of Cryptococcus albidus concentrate per 1 000 litres of water) for 2 minutes in a packhouse.

### Group 6

Pears were sprayed with the aqueous suspension (50 litres of Cryptococcus albidus concentrate per 1 000 litres of water) on a packing line by means of an atomizer.

Total sample size of the treated pears used in Trials A and B was 80 bulk bins of pears containing approximately 450 kilograms of pears and the control pears for each trial comprised 2 bulk bins.

Treated and control pears were packed in 15 kilogram export cartons and cold stored at - 0.5°C for 3 months before being transferred to 20°C for 7 days. After storage at 20°C, the number of pears with decay lesions was recorded and the fungal pathogen responsible for the decay identified. The sample size for the various treatments conducted for Trials A and B varied from 62 - 80 cartons per group.

The number of pears exhibiting decay lesions in treated samples drenched at the packhouse for Trial B, Group 5 was approximately 44% less than in the samples of control pears. Conditions of application were not ideal and it is believed that routine experimentation to find a suitable wetting and sticking agent can improve the effectiveness of application and enhance the effectiveness of decay control.

The results of the treatment of the various groups are set out in Table 1 below:

**TABLE 1:**

| Incidence of decay in pears treated with suspensions containing propagules of *Cryptococcus albidus* | | |
|---|---|---|
| TRIALS | Incidence of decay (%) | Reduction (%) |
| TRIAL A | | |
| Group 1 | 1,27 | 20,9 |
| Group 2 | 0,94 | 41,2 |
| Group 3 | 1,03 | 35,8 |
| TRIAL B | | |
| Group 4 | 1,60 | 0 |
| Group 5 | 0,90 | 43,9 |
| Group 6 | 1,29 | 19,7 |

### Ribosomal DNA fingerprinting of Cryptococcus albidus

### A. Yeast-cultivation/chromosomal DNA isolation and purification

A 10 ml yeast culture of Cryptococcus albidus (CBS accession No. 604.94) was prepared by inoculating rich YEPD medium (1 % yeast extract; 2 % peptone; 2 % glucose), and then incubating the culture for twenty four hours at 30°C on a rotary shaker. Chromosomal deoxyribonucleic acid (DNA) of the yeast was isolated and purified according to Hoffman and Winston (1987 - reference 1). Accordingly, in essence, the DNA isolation and purification was done as follows: The yeast cells were harvested by centrifugation, washed with distilled water and transferred to a microfuge tube. The yeast cells were broken with the aid of acid-washed glass beads in the presence *of* Triton X-100 and phenol/chloroform/isoamyl alcohol. A DNA/RNA (ribonucleic acid) fraction was separated from the cell debris by centrifugation and precipitated in the presence of 70 % ethanol. The RNA was removed from the fraction with the aid of RNase A enzyme (obtained from Boehringer Mannheim in South Africa) and the remaining DNA fraction was then purified through a subsequent ethanol precipitation step to obtain 5 *µ*g of purified chromosomal DNA. The chromosomal DNA was finally dissolved in 50 *µ*l Tris-EDTA buffer (TE) (Composition : 10 mM Tris-HCL and 1 mM ethylene-diamine-tetra acetic acid).

### B. Ribosomal DNA restriction enzyme analysis

(a) The purified chromosomal DNA (5 *µ*g) of the Cryptococcus albidus culture was subjected to restriction digestion at 37°C with BglII, EcoRI, PstI, PvuII, SalI, XbaI and XhoI for three hours respectively. The DNA was separated on horizontal 0.8 % agarose gels and transferred to Hybond-N nylon membranes (available from Amersham International plc, Amersham Place, Little Chalfont, Buckinghamshire HPC 9NA, United Kingdom) in accordance with the procedure of Sambrook et al. (1984-reference 2).
(b) The ribosomal DNA fragments on the nylon membranes were marked with a [A^{32P}] ATP radioactively labelled 6,3-bp Pst fragment of Neurospora crassa rDNA repeat, resident on plasmid pMF2 (Russell et al., 1984 - reference 3).
(c) Using the Neurospora crassa rDNA probe, seven distinct BglII, EcoRI. PstI. PvuII, SalI, Xbal and XhoI restriction patterns unique to this strain of Cryptococcus albidus, were obtained, (as set out in Table 2 below).

**Table 2 -**

| Ribosomal DNA fragments sizes (base pairs) | | | | | | |
|---|---|---|---|---|---|---|
| **BglII** | **EcoRI** | **PstI** | **PruII** | **SalI** | **XbaI** | **XhoI** |
| 3700 | 3800 | 5000 | > 15000 | ~10000 | 4000 | ~10000 |
| 2500 | 2600 | 3400 | | | 2000 | |
| 2350 | | | | | | |

This unique rDNA fingerprint identifies this strain of Cryptococcus albidus from other antagonistic yeast strains.

The present invention thus provides for the use of the yeast Cryptococcus albidus as a biocontrol agent for controlling or preventing the infection of fruit of plants by plant pathogens. In other words, the yeast strain can be used as an effective biological control agent for preventing or minimising post-harvest decay of fruit from plants. It is believed that the yeast strain inhibits or prevents plant pathogens from infecting fruit. The yeast strain acts as an antagonist to the plant pathogens. In other words, amensalism or antagonism of the plant pathogen or-pathogens by the yeast strain occurs, thereby preventing or minimising infection of fruit of plants by fungal pathogens.

### REFERENCES

### (These references are incorporated herein by reference thereto)

1. HOFFMAN, C.S. and WINSTON, F., 1987. A ten-minute DNA preparation from yeast efficiently releases autonomous plasmids for transformation of Escherichia coli. Gene 57 : 267-272.
2. SAMBROOK, J., FRITSCH, E.F. and MANIATIS, T., 1989. Molecular cloning : A laboratory manual. 2nd ed. Cold Spring Harbor New York, Cold Spring Harbor Laboratory Press.
3. RUSSELL, P.J., WAGNER, S., RODLAND, K.D., FEINBAUM, R.L., RUSSEL, J.P., BRET-HARTE, M.S., FREE, S.J. and METZENBERG, R.L., 1984. Organization of the ribosomal ribonucleic acid genes in various wild-type strains and wild-collected strains of Neurospora. Molecular and General Genetics 196 : 275-282.

## Claims

1. A method of controlling or preventing infection of fruit by plant pathogens, which method includes applying to fruit, or to at least a part of a plant bearing the fruit, an effective amount of biocontrol yeast strain of Cryptococcus albidus identified by Centraalbureau Voor Schimmelcultures (CBS) accession No. 604.94 which is non-pathogenic to the fruit and plant.

2. A method as claimed in claim 1, in which the yeast strain is applied in the form of an aqueous suspension having a concentration of 1 X 10⁴ - 1 X 10⁸ propagules/cm³.

3. A method as claimed in claim 1 or claim 2, in which the yeast strain is applied 7-2 days before harvesting of the fruit.

4. A method as claimed in claim 1 in which the yeast strain is applied to the fruit and optionally to other parts of plants at a fruit bearing stage, prior to harvesting.

5. A method as claimed in claim 3 or claim 4 in which the yeast strain is applied to the fruit and leaves and stems of the plant in proximity to the fruit.

6. A method as claimed in claim 1 or claim 2, in which the yeast strain is applied to the fruit after harvesting of the fruit by drenching the fruit in an aqueous suspension of the yeast strain.

7. A method as claimed in any one of the preceding claims, in which said plant pathogens are selected from the group consisting of Botrytis cinerea and Penicillium expansum and wherein the fruit is selected from the group consisting of apples and pears.

8. A biocontrol composition which comprises, as active biocontrol agent, an effective amount of a yeast strain of Cryptococcus albidus identified by CBS accession No. 604.94 in combination with a biologically acceptable carrier material.

9. A Cryptococcus albidus yeast strain identified by Centraalbureau Voor Schimmelcultures (CBS) accession No. 604.94.

## Patentansprüche

1. Verfahren zur Bekämpfung oder Prävention von Fruchtinfektion durch Pflanzenpathogene, wobei das Verfahren das Aufbringen auf die Frucht oder auf mindestens einen Teil einer Pflanze, die die Frucht trägt, einer wirksamen Menge eines auf biologische Weise kontrollierenden Hefestammes des *Cryptococcus albidus* umfaßt, identifiziert durch Hinterlegungsnummer 604.94, des Centraalbureau Voor Schimmelcultures (CBS), der nicht pathogen für Frucht und Pflanze ist.

2. Verfahren nach Anspruch 1, **dadurch gekennzeichnet, daß** der Hefestamm in Form einer wäßrigen Suspension mit einer Konzentration von 1 x 10⁴ - 1 x 10⁸ Vermehrungseinheiten/cm³ aufgebracht wird.

3. Verfahren nach Anspruch 1 oder 2, **dadurch gekennzeichnet, daß** der Hefestamm 7 bis 2 Tage vor Ernten der Frucht aufgebracht wird.

4. Verfahren nach Anspruch 1, **dadurch gekennzeichnet, daß** der Hefestamm auf die Frucht und optional auf andere Teile der Pflanzen in einem fruchttragenden Stadium aufgebracht wird, bevor geerntet wird.

5. Verfahren nach Anspruch 3 oder 4, **dadurch gekennzeichnet, daß** der Hefestamm auf die Frucht und Blätter und Stengel der Pflanze in der Nähe der Frucht aufgebracht wird.

6. Verfahren nach Anspruch 1 oder 2, **dadurch gekennzeichnet, daß** der Hefestamm auf die Frucht nach Ernten der Frucht durch Tränken der Frucht in einer wäßrigen Suspension des Hefestammes aufgebracht wird.

7. Verfahren nach einem der vorangehenden Ansprüche, **dadurch gekennzeichnet, daß** besagte Pflanzenpathogene ausgewählt werden aus einer Gruppe umfassend *Botrytis cinerea* und *Penicillium expansum* und die Frucht ausgewählt ist aus einer Gruppe umfassend Äpfel und Birnen.

8. Auf biologische Weise kontrollierende Zusammensetzung, die als aktives auf biologische Weise kontrollierendes Agens eine wirksame Menge des Hefestammes von *Cryptococcus albidus* umfaßt, identifiziert durch Hinterlegungsnummer 604.94, des CBS in Kombination mit einem biologisch akzeptablen Trägermaterial.

9. *Cryptococcus albidus*-Hefestamm, identifiziert durch Hinterlegungsnummer 604.94, des Centraalbureau Voor Schimmelcultures (CBS).

## Revendications

1. Procédé de lutte et de prévention de l'infection des fruits par des pathogènes des plantes, ledit procédé comprenant les étapes consistant à appliquer aux fruits, ou au moins à une partie de la plante portant les fruits, une quantité efficace de souche de levure de Cryptotoccus albidus agissant comme agent de contrôle biologique, identifiée par le N° d'accès du Centraalbureau Voor Schimmelcultures (CBS) 604.94, et étant non pathogène pour les fruits et pour la plante.

2. Procédé selon la revendication 1, dans lequel la souche de levure est appliquée sous la forme d'une suspension aqueuse ayant une concentration de 1 x 10⁴ - 1 x 10⁸ propagules/cm³.

3. Procédé selon la revendication 1 ou la revendication 2, dans lequel la souche de levure est appliquée 7 à 2 jours avant la cueillette des fruits.

4. Procédé selon la revendication 1, dans lequel la souche de levure est appliquée sur les fruits et éventuellement sur d'autres parties des plantes au moment où elles portent les fruits, avant la cueillette.

5. Procédé selon la revendication 3 ou la revendication 4, dans lequel la souche de levure est appliquée sur les fruits, les feuilles et les tiges de la plante à proximité des fruits.

6. Procédé selon la revendication 1 ou la revendication 2, dans lequel la souche de levure est appliquée sur les fruits après la cueillette des fruits en trempant les fruits dans une suspension aqueuse de la souche de levure.

7. Procédé selon l'une des revendications précédentes, dans lequel les pathogènes des plantes sont choisis parmi Botrytis cinerea et Penicillium expansum et dans lequel les fruits sont choisis parmi les pommes et les poires.

8. Composition de lutte biologique comprenant, comme agent de lutte biologique actif, une quantité efficace d'une souche de levure de Cryptococcus albidus identifiée par le N° d'accès CBS 604.94 en combinaison avec un support biologiquement acceptable.

9. Souche de levure de Cryptococcus albidus identifiée par le N° d'accès du Centraalbureau Voor Schimmelcultures (CBS) 604.94.
